# EUROPEAN PATENT SPECIFICATION

(11) **EP 1 666 057 B1**
(45) Date of publication and mention of the grant of the patent: **18.02.2009**
(21) Application number: 04018329.5
(22) Date of filing: 26.04.1990
(51) Int. Cl.: A61K 39/10, A61P 31/04

(54) **Method of manufacture of an acellular vaccine comprising Bordetella pertussis antigens**
Verfahren zur Herstellung eines azellulären Impfstoffes der Bordetella pertussis Antigene enthält
Procédé de production d'un vaccin acellulaire contenant des antigènes de Bordetella pertussis

(30) Priority: 08.05.1989 GB 8910570
(43) Date of publication of application: 07.06.2006
(62) Divisional of application: 96112324.7
(73) Proprietor: MEDEVA B.V., 1078 ED Amsterdam (NL)
(72) Inventor: Novotny, Pavel, deceased (NL)
(74) Representative: Campbell, Patrick John Henry

(56) References cited:
- EP-A- 0 162 639
- EP-A- 0 267 998
- M. DE MAGISTRIS ET AL.: "Dissecting human T cell responses against Bordetella species." THE JOURNAL OF EXPERIMENTAL MEDICINE, vol. 168, no. 4, October 1988 (1988-10), pages 1351-1362, XP002085627 New York, NY, USA
- M. BRENNAN ET AL.: "Structural and functional properties of a 69-kilodalton outer membrane protein of Bordetella pertussis." TOKAI JOURNAL OF EXPERIMENTAL AND CLINICAL MEDICINE, vol. 13, no. supplement, 1988, pages 211-215, XP008090707 Tokyo, Japan
- I. CHARLES ET AL.: "Molecular cloning and analysis of P.69, a vir-controlled protein from Bordetella pertussis." TOKAI JOURNAL OF EXPERIMENTAL AND CLINICAL MEDICINE, vol. 13, no. supplement, 1988, pages 227-234, XP008090706 Tokyo, Japan
- "Placebo-controlled trial of two acellular pertussis vaccines in Sweden - protective efficacy and adverse events." THE LANCET, vol. 1, no. 8592, 30 April 1988 (1988-04-30), pages 955-960, XP009072139 London, UK
- P. NOVOTNY ET AL.: "Bordetella specific protective antigen." Abstract disclosed at the International Workshop on Bordetella Pertussis, 18-20 August 1988, Rocky Mountain Laboratories, Hamilton, Montana, USA

## Description

The present invention relates to methods of manufacture of acellular Bordetella pertussis vaccine compositions, in particular an acellular Bordetella pertussis vaccine comprising a synergistic combination of the 69kDa antigen and the filamentous haemagglutinin antigen (FHA) from B. pertussis.

Bordetella pertussis causes a serious and debilitating disease in.humans, children being particularly susceptible, which is kept under control in the developed countries by large scale immunisation programmes. It has been found that immunisation is a very important factor in the reduction of the disease and that failure to vaccinate can lead to increased incidence of the disease. In practically all areas, immunisation is effected using a whole cell B.pertussis vaccine which has been found to be relatively effective in preventing the disease. However, recently, concern over adverse reactions to the vaccine has led to lower vaccine acceptance and debate about its continued use. Some of the adverse reactions noted include fever, local reactions and persistant screaming. The incidence of fever and persistant screaming have been estimated to occur in 7% of patients (Wardlaw et al Medical Microbiology Vol.2. Immunisation against Bacterial Disease 1983).

With the currently low occurrence of the disease in developed countries with immunisation programmes, the benefit/risk ratio is poorly defined, and many clinicians believe that the risk of inoculation outweighs the benefits gained by immunisation. As a result, many children are not inoculated and there is now a consequent risk of a pandemic of whooping cough. Indeed in recent years the incidence of whooping cough and resulting infant morbidity has increased as the use of the whole cell vaccine has decreased. Considerable research effort has, therefore, been directed towards the development of improved pertussis vaccines and especially acellular vaccines which lack the components associated with the toxic effects of the whole cell vaccines which have caused the concerns, whilst incorporating those components necessary to protect against the disease. Examples of such efforts can be found in De Magistris et al (1988) The Journal of Experimental Medicine, 168(4), 1351-1362; EP-A-0267998 (Institut Pasteur); and Novotny et al, abstract entitled "Bordetella specific protective antigen", International Workshop on Bordetella pertussis, August 1988, Rocky Mountain Laboratories, Hamilton, Montana.

The search for a safer, effective, acellular B.pertussis vaccine has been hampered in the past by the paucity of information regarding the identity and mechanisms of action of the pathogenic, toxic and protective moieties of B, pertussis contained in the whole cell vaccines. Work has, therefore, been concentrated on isolating and purifying the 20 or more surface antigens of the B.pertussis organism and characterising their ability to induce immune reactions (see, for example. González J. Am.Med.Soc. 248 (1) 22-23). Examples of antigens that have been suggested for investigation include lymphocytosis promoting factor (pertussis toxin/LPF), filamentous haemagglutinin (FHA), lipopolysaccharide (LPS), agglutinogens, dermonecrotic toxin (DNT), heat labile and heat stable toxins, polymorphonuclear leukocyte inhibitor factor, adenylate cyclase and other surface components. Other proposed candidate antigens for investigation include tracheal cytotoxin and various outer membrane proteins.

An early extract vaccine was developed by L.Pillemer (Proc.Soc.Exp.Biol.Med. (1950) 75, 704-705) which was based on disrupted B.pertussis cells and found to provide protection, but which was not adopted commercially in view of the toxicity of the preparation.

Examples of more recent B,pertussis extract vaccines that have been suggested include those described in UK Patent Specification 2 083 358A (Takeda) involving removal of endotoxin from culture supernatants; French Patent Specification 2 047 886 (Institut Merieux) involving extraction of a microbial suspension with an anionic surfactant; and Japanese Patent Specification 58-222032 (Teijin) which comprises a sub-unit protein vaccine based on pertussis toxin (LPF).

Much of the work carried out on acellular pertussis vaccines is concentrated on the possibility of basing such a vaccine on LPF. However, it is believed that some of the adverse effects hitherto observed to be associated with pertussis vaccination are related to the toxin. In combination with tetanus or diphtheria toxoid and LPS, it is able to induce experimental encephalopathy in susceptible mice (L.Steinman, et al. Nature (1982) 299, 738-740; Redhead et al, Workshop on B, pertussis, Nat. Inst. of Biol. Standards & Controls, Holly Hill, Hampstead, London, 1983). Thus some clinicians believe that LPF may, possibly, be responsible for brain damage should such complications occur after vaccination.

Nonetheless, studies to date, have generated data which has led to a general belief that LPF is an essential part of any acellular vaccine (Bacterial Vaccines, 1984, Chapter 3, Manclark et al, Editor Germanier).

A new acellular vaccine, currently available in Japan has been tested in controlled clinical trials in Sweden. This vaccine includes the pertussis toxin (LPF) and FHA or LPF alone (Lancet 1 955, 1988). However, this vaccine has proved not to be as effective as a whole cell vaccine, providing only about 69% protection.

Apart from the poor protective effect, three deaths occurred in the toxin-based vaccine group which may possibly be associated with the vaccine. Considering all these data, the Swedish Health Authority refused to licence this so called "Japanese Vaccine" in Sweden.

This clinical trial, however, is an illustration of the belief that LPF antigen is an essential component of the vaccine since it has been suggested that whooping cough is a toxin-mediated disease and that the protection of mice in the pertussis mouse protection test is solely dependent on the presence of an active LPF in the preparation (Pittman, M. 1984 : The Concept of Pertussis as a Toxin-Mediated Disease, Pediatric Infections Disease, 3, 467-486). It is believed that these assumptions are incorrect.

Filamentous haemagglutinin (FHA) is a protein having a molecular weight of 107-130 kDa and appears as filaments in the electron microscope. It is a haemagglutin that is inhibited by cholesterol.

Many research groups have suggested that FHA may be an important immunogen and vaccine candidate. (For a review see Bacterial Vaccines 1984, Chapter 3, Manclark et al, Editor Germanier). Our data shows, however, that FHA alone only provides minimal protection.

The 69kDa antigen of pertussis is an outer membrane protein, is heat-stable and can be prepared by methods known in the art (see EP0162639). The use of 69kDa on its own is not as efficient as the whole cell vaccine.

The present inventor has found that a combination of 69kDa and FHA together is, surprisingly, more potent than the aggregate effect of the individual components. The synergistic combination of 69kDa and FHA is advantageous since LPF is not required, and consequently the chances of adverse effects are reduced. Additionally, a bivalent vaccine containing only 69kDa and FHA will clearly be easier and cheaper to manufacture than a trivalent vaccine containing LPF as well.

Apart from that, by a proper combination of pertussis antigens the equal effective dose of the suggested combination is up to 15 times lower than, for example, a combination of the 69kDa protein and LPF.

Thus, according to the present invention there is provided a method for the preparation of an acellular vaccine, which method comprises preparing the 69kDa antigen of Bordetella pertussis as an individual component, preparing the filamentous haemagglutinin antigen of Bordetella pertussis as an individual component, and mixing the 69kDa antigen and the filamentous haemagglutinin antigen in amounts that provides the 69kDa antigen and the filamentous haemagglutinin antigen in a weight ratio of between 1:10 and 1:1, so as to produce a synergistic effect in vaccine potency.

The method provides a synergistic combination comprising i) the 69kDa antigen from B.pertussis and ii) the filamentous haemagglutinin antigen of B. pertussis in an amount effective to induce protection in a mammal to subsequent challenge by a virulent strain of B. pertussis.

The ratio of 69kDa antigen to FHA is preferably approximately 1:1.

The 69kDa and FHA antigens may be concomitantly used for the prophylatic treatment of mammals susceptible to B. pertussis infections.

Pharmaceutically acceptable excipients may be included in the vaccines and may be liquid media suitable for use as vehicles to introduce the antigens into the patient. An example of such a carrier is saline solution. The antigenic proteins may be in solution or suspended as a solid in the carrier.

The vaccine formulation may also comprise an adjuvant for stimulating the immune response and thereby enhancing the effect of the vaccine. Convenient adjuvants for use in the present invention include, for example, aluminium hydroxide and aluminium phosphate.

Conveniently the vaccine formulations are presented to contain a final concentration of antigenic protein in the range of from 0.01 to 5 mg/ml, preferably 0.03 to 2 mg/ml, most preferably 0.3 mg/ml. After formulation the vaccine may be incorporated into a sterile container which is then sealed and stored at a low temperature, for example 4°C, or may be freeze-dried.

In order to induce immunity in man to whooping cough one or more doses of the vaccine suitably formulated may be administered. It is recommended that each dose is 0.1 to 2 ml, preferably 0.2 to 1 ml, most preferably 0.5 ml of vaccine.

The use of 69kDa and FHA provides a vaccine for use in the induction of immunity to whooping cough in man. The vaccines may be administered by any conventional method for the administration of vaccines including oral and parenteral. The treatment may consist of a single dose or a plurality of doses over a period of time.

Accordingly, there is disclosed a method of treatment of a mammal susceptible to B. pertussis infections comprising the administration concurrently of 69kDa antigen of B.pertussis and FHA.

### Example 1

### Preparation of Filamentous Haemagglutinin (FHA)

FHA can be prepared in methods well known in the art (see Arai and Munoz J.J. (1979), Infection and Immunity 25 764-767; Ashworth et al (1982) Infect. Immun 37 1278-1281; Cowell et al Bacterial Vaccines, p371-379 Seminars in Infectious diseases Vol. IV (1982); Sato et al (1983) Infect. Immun 41 313-320). However FHA in the following procedure was prepared in accordances with the following protocol.

FHA purification: B.pertussis Tomaha or BP357 (Tn5 transposon mutant of AA.Weiss et al (1983) which does not secrete the LPF) or W28ΔLPF obtained from R.Rappuloi were grown in Stainer & Scholte medium (0.05 Tris) in 650ml Costar flasks (150ml in each) for 5 days at 3.7°C (Sato et al 1983 supra). Before centrifugation (30mins at 6000 x g) 50µM 1,10-phenanthroline monohydrate as proteolysis inhibitor was added to the cultures. The cell free supernatant adjusted to pH 8.7 (using 5N.NaOH) was applied to a 30 x 350mm column of Spheroidal Hydroxylapatice (BHD) at a flow rate of 500ml/hr. (All operations at room temperature) . The column was then washed in the cold room (+4°C) until stable baseline was achieved at a flow rate of 50ml/hr with (a) 10mM phosphate buffer, pH 8.0, (b) 100mM phosphate buffer, pH 8.0, and finally (c), the retained material was eluted with 0.5 N.NaCl in 100mM phosphate buffer, pH 7.0. The peak fractions agglutinating goose red blood cells (10µl volumes from each fraction suspended in 50ul of PBS and an equal amount of washed 0.5% goose blood cells were added and incubated for 1-2 hours at 37°C) were pooled. The Pool was dialyzed overnight against 20-30 volumes of 0.025M Bis-Tris/HCl buffer, pH 7.1, at 4°C. The precipitated FHA was collected on a centrifuge (20mins at 8000xg). The next step was inspired by Cowell et al (1983) who found that the FHA (as well as LPF) is soluble in 40mM beta-alanine buffer, pH 3.5. The precipitated FHA was solubilized in the smallest possible volume of β-alanine buffer (3.57g β-alanine and 0.35g formic acid per litre), insolubles removed by centrifugation and the clear supernatant was applied to a column (25 x 800mm) of Ultrogel AcA 34 or Aca 44 equilibrated and eluted with the same buffer to remove impurities. The retained haemagglutinating material appeared in a peak eluted by 0.05M.Tris/HCl buffer containing 0.5M NaCl (pH7.2). The fractions from the main peak and having haemagglutinatious properties were pooled and kept frozen or re-precipitated by dialysis against 0.025M Bis-Tris/HCl buffer, pH 7.1 and dissolved in a smaller volume of β-alanine buffer. The solubility is approx 3.0mg FHA/ml. The final product thus obtained, either from the , Tomaha, BP357 or W28 LPF strain does not contain detectable amount of LPF as measured by CHO cell assay (which was negative at a concentration of 2-3ug FHA per single well containing 200ul tissue culture; sensitivity of the test: 1-2pg LPF per well gives a positive clumping) or by histamine sensitization. The N:NIH-S mice were injected with doses of 50ug of FHA intreperitoneally and challenged 4 days later by intraperitoneal injection if 4mg histamine hydrochloride. None of them died. The material frozen (at -20 or -40°C) at acid pH appears stable as judged from its ELISA reactivity and appearance in the SDS-PAGE: It forms prevalently three strong bands in the region of 150-100 kD.

### Example 2

69kDa antigen was prepared in accordance with the procedures outlined in published European patent application No. 0162 639, using either strain BP357 or W28ΔLPF of B.pertussis, and immunopurified using the monoclonal antibody BB05 - deposited at the Public Health Laboratory Service Centre for Applied Microbiology and Research, Porton Down, Salisbury Wiltshire SP4 OJG United Kingdom, under No 90020103 on 1st February 1990.

### Example 3

Kendrick test. This was performed according to W.H.O. Requirements for Pertussis Vaccine using outbred NIH-S mice (OLAC, category 3, free of most pathogens including B.bronchiseptica), weighting 14-16g. The antigens, in 0.5ml volumes, were inoculated intraperitoneally as a mixture, and comprised a top dilution and three three-fold serial dilutions. After two weeks the mice were challenged intracerebrally using the recommended challenge strain 18-323 (-400 LD₅₀). The number of survivors in each group was used for calculation of the relative potency in respect to the British Pertussis Reference Vaccine 66/84 using a program of parallel line probit analysis. A comparative test was also preformed using an 69kDa/LPF vaccine. The results are shown in Table 1. In this experiment the 69kDa protein and FHA originated from the strain BP357.

**TABLE 1**

| 69kd (µg) | FHA (µg) | SURVIVORS |
|---|---|---|
| 20 | 20 | 13/16 |
| 6.7 | 6.7 | 12/16 |
| 2.2 | 2.2 | 8/16 |
| 0.74 | 0.74 | 8/16 |
| | | |
| 69kd (µg) | LPF (ng) | |
| 20 | 100 | 8/16 |
| 6.7 | 33 | 6/16 |
| 2.2 | 11 | 0/16 |
| 0.74 | 3.7 | 1/16 |
| | | |
| 66/84 | I.U. | |
| Whole cell UK | 0.25 | 9/15 |
| reference | 0.08 | 9/16 |
| vaccine | 0.028 | 2/16 |
| | 0.009 | 1/16 |

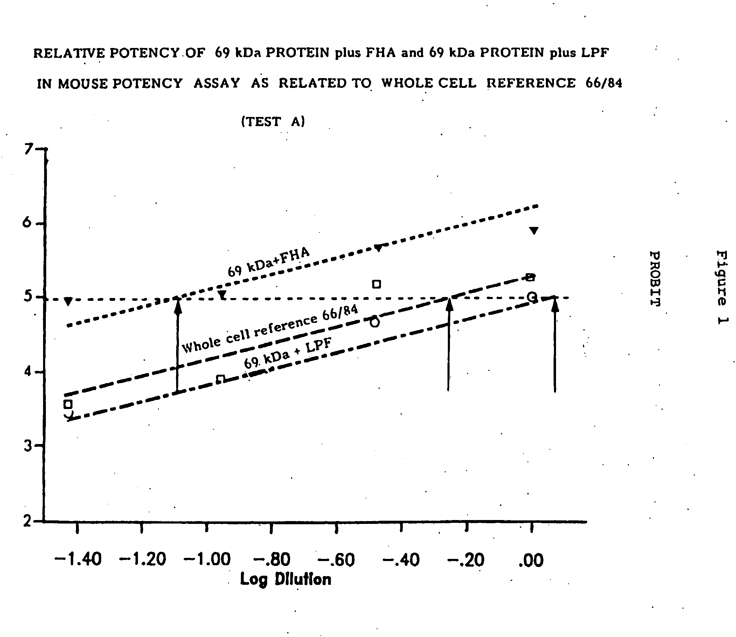

This data was computed according to the methodology of parallel line probits analysis. The results are displayed in Figure 1.

The results clearly show that the 69kDa/FHA vaccine is more potent than 69kDa/LPF and at least as, if not more, potent than the whole cell vaccine.

### Example 4

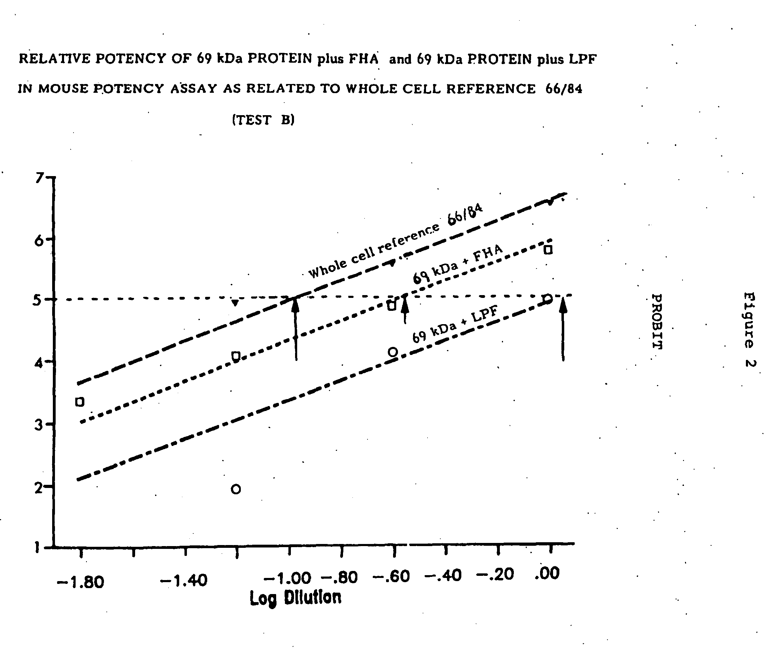

Combinations and/or individual antigens originating from the strain W28ΔLPF of Bordetella pertussis were used in another Kendrick test. Since in this strain the whole toxin gene has been blocked, the possibility that these preparations were contaminated by LPF is nil. The antigen(s) in 0.5ml volumes were inoculated intraperitoneally, individually or as mixtures and comprised a top dose and three four-fold dilutions. After two weeks the mice were challenged intracerebrally using a challenge strain 18-323 (ca 400 LD50). The number of survivors in each group was used for the calculation of the relative potency in respect of the British Pertussis Reference Vaccine 66/84 using a program of parellel line probit analysis. The British Reference 66/84 was used at top dilution containing 0.5 I.U. and three four-fold serial dilutions. The results are shown in table 2 and figure 2.

**TABLE 2**

| 69kDa (ug) | FHA (ug) | LPF (ng) | SURVIVORS/TOTAL |
|---|---|---|---|
| 20.0 | - | - | 0/18 |
| 5.0 | - | - | 1/18 |
| 1.25 | - | - | 1/18 |
| 0.325 | - | - | 0/18 |
| 20.0 | - | 100.0 | 8/17 |
| 5.0 | - | 20.0 | 3/17 |
| 1.25 | - | 5.0 | 0/20 |
| 0.325. | - | 1.25 | 0/18 |
| 20.0 | 20.0 | - | 14/18 |
| 5.0 | 5.0 | - | 8/18 |
| 1.25 | 1.25 | - | 3/18 |
| 0.325 | 0.325 | - | 1/18 |
| - | 20.0 | - | 0/17 |
| - | 5.0 | - | 6/18 |
| - | 1.25 | - | 4/18 |
| - | 0.325 | - | 1/17 |
| Whole cell reference 66/84 | | | |
| 0.5 I.U. | | | 16/17 |
| 0.125 | | | 13/18 |
| 0.031 | | | 8/17 |
| 0.008 | | | 0/18 |
| Challenge titration: | | | |
| Challenge dose 0/11 | | | |
| " | " 1/50 | | 1/10 |
| " | " 1/250 | | 2/10 |
| " | " 1/1250 | | 6/9 |

## Claims

1. A method for the preparation of an acellular vaccine, which method comprises preparing the 69kDa antigen of Bordetella pertussis as an individual component, preparing the filamentous haemagglutinin antigen of Bordetella pertussis as an individual component, and mixing the 69kDa antigen and the filamentous haemagglutinin antigen in amounts that provide the 69kDa antigen and the filamentous haemagglutinin antigen in a weight ratio of between 1:10 and 1:1, so as to produce a synergistic effect in vaccine potency.

2. A method according to claim 1wherein the vaccine is devoid of the B. pertussis toxin.

3. A method according to claim 1 or 2 wherein the 69kDa antigen and the filamentous haemagglutinin antigen are provided in a weight ratio of approximately 1:1.

4. A method according to claim 1, 2 or 3 wherein the antigenic protein is provided in a concentration in the range of from 0.01 to 5.0 mg/ml.

## Patentansprüche

1. Methode zur Präparation eines azellulären Impfstoffs, welche Methode das Präparieren eines 69 kDa-Antigens von Bordetella pertussis als einer einzelnen Komponente, das Präparieren des filamentösen Hämagglutinin-Antigens von Bordetella pertussis als einer einzelnen Komponente und das Mischen des 69 kDa-Antigens und des filamentösen Hämagglutinin-Antigens in Mengen umfasst, die das 69 kDa-Antigen und das filamentöse Hämagglutinin-Antigen in einem Gewichtsverhältnis von zwischen 1:10 und 1:1 bereitstellen, um eine synergistische Wirkung bei der Impfstoffpotenz zu erzeugen..

2. Methode nach Anspruch 1, wobei der Impfstoff frei von dem B. pertussis-Toxin ist.

3. Methode nach Anspruch 1 oder 2, wobei das 69 kDa-Antigen und das filamentöse Hämagglutinin-Antigen in einem Gewichtsverhältnis von etwa 1:1 bereitgestellt werden.

4. Methode nach Anspruch 1, 2, oder 3, worin das antigene Protein in einer Konzentration in dem Bereich von 0,01 bis 5,0 mg/ml bereitgestellt wird.

## Revendications

1. Procédé pour la préparation d'un vaccin acellulaire, lequel procédé comprend la préparation de l'antigène de 69 kDa de *Bordetella pertussis* sous la forme d'un constituant individuel, la préparation de l'antigène hémagglutinine filamenteuse de *Bordetella pertussis* sous la forme d'un constituant individuel, et le mélange de l'antigène de 69 kDa et de l'antigène hémagglutinine filamenteuse en des quantités qui procurent l'antigène de 69 kDa et l'antigène hémagglutinine filamenteuse en un rapport pondéral compris entre 1:10 et 1:1, de façon à produire un effet synergique quant à la puissance vaccinale.

2. Procédé selon la revendication 1, dans lequel le vaccin est dépourvu de la toxine de *B. pertussis*.

3. Procédé selon la revendication 1 ou 2, dans lequel l'antigène de 69 kDa et l'antigène hémagglutinine filamenteuse sont présents en un rapport pondéral d'approximativement 1:1.

4. Procédé selon la revendication 1, 2 ou 3, dans lequel la protéine antigénique est présente en une concentration comprise entre de 0,01 et 5,0 mg/ml.
